# EUROPEAN PATENT APPLICATION

(11) **EP 2 236 112 A1**
(43) Date of publication of application: **06.10.2010**
(21) Application number: 08870193.3
(22) Date of filing: 20.11.2008
(51) Int. Cl.: A61F 13/15, A61F 13/49, A61F 13/494, A61F 13/511

(54) **ABSORBENT ARTICLE**

(30) Priority: 11.01.2008 JP 2008004892
(71) Applicant: Unicharm Corporation, Ehime 799-0111 (JP)
(72) Inventor: NAKAJIMA, Kaiyo, Kanonji-shi Kagawa 769-1602 (JP); MINATO, Hironao, Kanonji-shi Kagawa 769-1602 (JP)
(74) Representative: Stark, Gordon Drummond
(86) International application number: PCT/JP2008/071076
(87) International publication number: WO 2009/087823

(57) **Abstract**

The present invention aims to restrict undesirable irritation to the wearer's skin, on one hand, and to protect the wearer's skin from contamination with body waste, on the other hand.

The diaper is provided on a liquid-absorbent panel (23) with a barrier sheet (3) and leak-barrier cuffs (4) both extending in a longitudinal direction (Y). The leak-barrier cuffs (4) raise themselves apart from the liquid-absorbent panel (23) and the barrier sheet (3) is pulled upward as well as outward in a transverse direction (X) under contraction of cuff-biasing elastic members (50). Front joint zones extend from a front end (27) to points lying aside from a front center line (r-r) toward the middle zone (32) and front segments (39a, 40a) of first and second barrier sheet-biasing elastic members (39, 40) have front maximally spaced points (41, 42) to define the maximum distance (W2) between the points (41, 42). Rear joint zones extend from a rear end (28) to a rear center line (s-s) and rear segments (39b, 40b) of the respective elastic members (39, 40) have rear maximally spaced points (43, 44) between which the rear segments (39b, 40b) are maximally spaced from each other.

## Description

### TECHNICAL FIELD

The present invention relates generally to absorbent articles and more particularly to such absorbent articles as disposable diapers, toilet training pants or incontinent briefs.

### RELATED ART

Disposable diapers to protect a wearer's skin from being soiled with body waste are known, for example, from JP1997-510385T. JP1997-510385T discloses a pants-type disposable diaper. This diaper is provided on an inner side of a pants-shaped cover sheet with a liquid-absorbent structure which is, in turn, provided with a pair of sheet-like flaps spaced from each other in a transverse direction. These flaps are contiguous to each other by the intermediary of a joint so that these flaps cooperate with the joint to form a front through-hole and a rear through-hole. These flaps are respectively provided along these front and rear through-holes with elastic members extending in a longitudinal direction and attached thereto under tension. Upon contraction of these elastic members, the joint is spaced apart from the liquid-absorbent structure so as to form below the front through-hole a pocket adapted to receive urine and under the rear through-hole a pocket adapted to receive feces.
PATENT DOCUMENT 1: JP1997-510385T

### DISCLOSURE OF THE INVENTION

### PROBLEM TO BE SOLVED BY THE INVENTION

The diaper of such type is put on a wearer's body with his or her external genital opposed to a front through-hole and anus opposed to a rear through-hole. For the diaper, it is generally desired to dimension the front through-hole's available open area as large as possible to prevent the peripheral edge thereof from coming in contact with the wearer's skin and to dimension the rear through-hole's available open area as small as possible in order to protect the wearer' buttock from coming in contact with body waste. While the peripheral edge of the front through-hole coming in contact with the wearer's external genital may sometimes irritate the wearer's skin and cause skin trouble, such skin trouble is caused often due to the elastic members attached to the peripheral edge of the through-hole. These elastic members attached to the sheet-like flaps assist the flaps to be moved upward apart from the liquid-absorbent structure and to reliably maintain the through-hole in its opened state while the elastic members come in contact with the skin with interposition of the sheet material forming the flaps. The flaps in themselves are sufficiently soft to avoid occurrence of the skin trouble due to irritation of the wearer's skin even the flaps come in contact with the wearer' skin. However, the elastic members attached to the flaps make sheet material of the flaps relatively stiff and, consequently, undesirable irritation of the wearer's skin will become serious when the elastic members come in contact with the wearer's skin with interposition of the sheet material forming the flaps.

In the case of the diaper disclosed in JP1997-510385T, the front through-hole's available open area is substantially the same as the rear through-hole's available open area and an included angle defined by the elastic members extending along the front through-hole is substantially the same as an included angle defined by the elastic members along the rear through-hole. With such diaper, if it is tried to enlarge the front through-hole's available open area in order to avoid any skin trouble of the wearer's skin, the rear through-hole' s available open area also will be inevitably enlarged. As a result, protection of the wearer's buttock from contact with body waste will be no more possible. Inversely, if it is tried to reduce the rear through-hole's available open area in order to protect the wearer's buttock from contact with body waste, the front through-hole's available open area also will be necessarily reduced, making it impossible to avoid any skin trouble occurring on the wearer's external genital.

In view of the problem as has been described above, it is an object of the present invention to provide an absorbent article improved so as to restrain skin irritation experienced by the wearer and to protect the wearer's skin from contact with body waste.

### MEASURE TO SOLVE THE PROBLEM

According to the present invention, there is provided an absorbent article having a longitudinal direction and a transverse direction, a side facing a wearer's skin and a side facing a garment, a front waist region, a rear waist region and a crotch region extending between the front and rear waist regions, and comprising a chassis provided in the crotch region with a liquid-absorbent structure and a barrier sheet provided on the side of the chassis facing the wearer's skin and movable apart from the chassis in the crotch region.

According to the present invention, the barrier sheet has lateral zones extending in the longitudinal direction and opposed to each other in the transverse direction, a middle zone by the intermediary of which the lateral zones are contiguous to each other, a front through-hole formed in the front waist region so as to be bordered by the middle zone, a rear through-hole formed in the rear waist region so as to be bordered by the middle zone, front and rear ends extending in the transverse direction and opposed to each other in the longitudinal direction, and first and second barrier sheet-biasing elastic members opposed to each other in the transverse direction and extending in the longitudinal direction and attached under tension to the barrier sheet in a substantially symmetric relationship about a longitudinal center line bisecting a dimension of the barrier sheet in the transverse direction. The front and rear ends are permanently bonded to the side of the chassis facing the wearer's skin and a void space is formed between the barrier sheet and the liquid-absorbent structure as the liquid-absorbent structure bows from the crotch region toward the front and rear waist regions, and the front and rear through-holes are defined by the lateral zones and the middle zone and adapted to guide body waste into the void space. The first and second barrier sheet-biasing elastic members comprise front segments extending along the front through-hole and rear segments extending along the rear through-hole and, on the assumption that the front and rear waist regions and the crotch region are forced into a substantially coplanar relationship, the front segments of the first and second barrier sheet-biasing elastic members are maximally spaced from each other in the transverse direction in a region defined aside from a front center line bisecting a dimension of the front through-hole in the longitudinal direction toward the middle zone, and the rear segments are maximally spaced from each other in the transverse direction in a region defined aside from a rear center line bisecting a dimension of the rear through-hole in the longitudinal direction toward the rear end.

According to one preferred embodiment of the present invention, the absorbent article further comprises a pair of leak-barrier cuffs provided on the side of the barrier sheet facing the wearer's skin so as to extend in the longitudinal direction and to be opposed to each other in the transverse direction. Each of the leak-barrier cuffs has a fixed edge joined to the chassis, a free edge not joined to the chassis so as to be movable apart from the chassis and cuff-biasing elastic member attached under tension to the free edge and causing the leak-barrier cuff to be spaced from the chassis. The barrier sheet and the leak-barrier cuffs are joined together in front joint zones lying between the fixed edges and the free edges opposed one to another in the transverse direction and overlapping the front segments of the first and second barrier sheet-biasing elastic members and the front joint zones extend intermittently or continuously from the front end of the barrier sheet to the points at which the first and second barrier sheet-biasing elastic members are maximally spaced from each other in the transverse direction.

According to another preferred embodiment of the present invention, the barrier sheet and the leak-barrier cuffs are jointed together at the rear end of the barrier sheet by means of rear joint zones and the rear segments of the first and second barrier sheet-biasing elastic members are joined together in the vicinity of the rear end of the barrier sheet so that the first and second barrier sheet-biasing elastic members may be maximally spaced from each other in the transverse direction in these joint zones.

According to still another preferred embodiment of the present invention, a dimension measured from the middle zone to a straight line connecting points at which front segments of the first and second barrier sheet-biasing elastic members are maximally spaced from each other is set to be shorter than a dimension measured from the middle zone to a straight line connecting points at which rear segments of the first and second barrier sheet-biasing elastic members are maximally spaced from each other.

### EFFECT OF THE INVENTION

According to the present invention, the front segments of the first and second barrier sheet-biasing elastic members are maximally spaced from each other at the region lying aside from the front center line bisecting the dimension of the barrier sheet toward the middle zone. Such unique construction makes it possible for the front segments to broaden the open area of the front through-hole and thereby to prevent the front through-hole from irritating the wearer's external genital and causing skin trouble. The rear segments of the first and second barrier sheet-biasing elastic members are maximally spaced from each other at the region lying aside from the rear center line toward the rear end. Such unique construction makes it possible for the rear segments to limit the open area of the rear through-hole on the side of the middle zone to an acceptable area and thereby to protect the wearer's buttock from contact with body waste. In other words, layout of the elastic members may be differentiated between the front and rear through-holes to restrict the skin trouble without affecting the characteristics of the front and rear through-holes.

The leak-barrier cuffs are provided on the side of the barrier sheet facing the wearer's skin so as to overlap the lateral zones of the barrier sheet, respectively, and the barrier sheet is joined to the leak-barrier cuffs in the front joint zones overlapping the front segments of the first and second barrier sheet-biasing elastic members. Such unique construction causes the lateral zones of the barrier sheet to be pulled outward as the leak-barrier cuffs move apart from the chassis. In this way, it is possible to broaden the open area of the front through-hole and thereby to alleviate irritation to the wearer's external genital.

The barrier sheet, the leak-barrier cuffs and the first and second barrier sheet-biasing elastic members are joined together in the vicinity of the rear end of the barrier sheet and, in these joint zones, the first and second barrier sheet-biasing elastic members are maximally spaced from each other in the transverse direction. Such unique construction restricts the movement in the transverse direction in the vicinity of the rear end. Consequentially, the lateral zones of the barrier sheet along which the first and second barrier sheet-biasing elastic members are attached are shape-stabilized, in other words, the inner edges of the rear through-hole defined by the lateral zones are position-stabilized. In the vicinity of the middle zone, the rear through-hole is substantially free from affection of the leak-barrier cuffs being spaced from the barrier sheet so that the distance between the first and second barrier sheet-biasing elastic members can be maintained relatively small. The open area of the rear through-hole would not be unacceptably broadened and, as an advantageous result, contact of the wearer's skin with body waste can be effectively alleviated.

The dimension measured from the middle zone to the straight line connecting the front maximally spaced points is shorter than the dimension measured from the middle zone to the straight line connecting the rear maximally spaced points. Such unique construction makes it possible to differentiate the pattern of the first and second barrier sheet-biasing elastic members between the front through-hole and the rear through-hole so that, in the front through-hole, the first and second barrier sheet-biasing elastic members may be maximally spaced from each other in the vicinity of the middle zone and these elastic members are prevented from coming in contact with the wearer's external genital opposed to this through-hole. In this way, the front through-hole is prevented from irritating the wearer's skin and causing the skin trouble.

### BRIEF DESCRIPTION OF THE DRAWINGS

[FIG. 1] Perspective view of the diaper according to a first embodiment.
[FIG. 2] Sectional view taken along the line II-II in Fig. 1.
[FIG. 3] Flatly developed plan view of a diaper shown by Fig. 1.
[FIG. 4] Explanatory diagram of an important part of Fig. 3.
[FIG. 5] Explanatory diagram of the important part of Fig. 3.
[FIG. 6] Sectional view taken along the line VI-VI in Fig. 4.
[FIG. 7] Explanatory diagram of the important part of the diaper according to a second embodiment.

### IDENTIFICATION OF REFERENCE NUMERALS USED IN THE DRAWINGS

- 1: diaper
- 2: chassis
- 3: barrier sheet
- 4: leak-barrier cuffs
- 5: inner side facing wearer's skin
- 6: outer side facing wearer's clothes
- 7: front waist region
- 8: rear waist region
- 9: crotch region
- 10: liquid-absorbent structure
- 27: front end
- 28: rear end
- 29: void space
- 30: lateral zone
- 31: lateral zone
- 32: middle zone
- 33: front through-hole
- 34: rear through-hole
- 39: first barrier sheet-biasing elastic members
- 40: second barrier sheet-biasing elastic member
- 41: front maximally spaced point
- 42: front maximally spaced point
- 43: rear maximally spaced point
- 44: rear maximally spaced point
- 50: cuff-biasing elastic members
- 53: front joint zone
- 54: rear joint zone

### DESCRIPTION OF THE BEST MODE FOR WORKING OF THE INVENTION

Embodiments of the present invention will be described on the basis of a disposable diaper as one example of disposable articles.

### <First Embodiment>

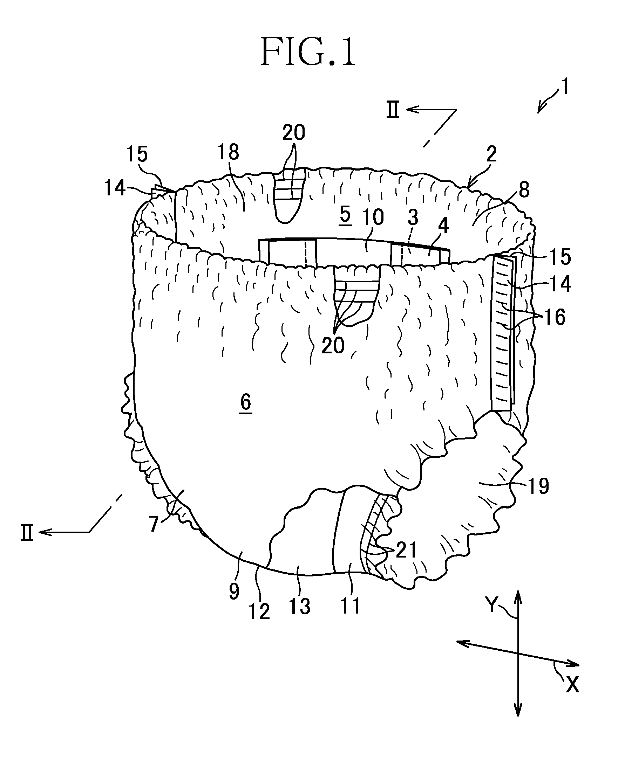

Fig. 1 illustrates a diaper 1 as put on the wearer's body. As illustrated, the diaper 1 comprises a liquid-absorbent chassis 2, a barrier sheet 3 and leak-barrier cuffs 4. The chassis 2 is pants-shaped and comprises an inner side 5 facing the wearer's skin, an outer side 6 facing the wearer's clothes, a front waist region 7, a rear waist region 8 and a crotch region 9 extending between these front and rear waist regions 7, 8. A direction extending from the front waist region 7 across the crotch region 9 toward the rear waist region 8 is referred to herein as a longitudinal direction Y and a direction orthogonal to this is referred to herein as a transverse direction X. The chassis 2 comprises an innerside sheet 11, an outerside sheet 12 and a liquid-impervious leak-barrier sheet 13 sandwiched between these innerside and outerside sheets 11, 12.

Transversely opposite side edges 14 of the front waist region 7 and side edges 15 of the rear waist region 8 associated with those of the front waist region 7 are put flat and joined together along arrays of joints 16 arranged intermittently along the respective side edges 14, 15 so as to form seams. The respective side edges 14, 15 of the front and rear waist regions 7, 8 are joined together along the respective arrays of joints 16 in this manner and thereby a waist-opening 18 is defined by the front and rear waist regions 7, 8 surrounding this and a pair of leg-openings 19 are defined by the respective arrays of seams 16 and the crotch region 9 cooperating to surround them. Along a peripheral edge of the waist-opening 18, a plurality of waist-surrounding elastic members 20 extend and along peripheral edges of the respective leg-openings, a plurality of leg-surrounding elastic members 21 extend. These elastic members 20, 21 are sandwiched between the innerside and outerside sheets 11, 12 and bonded under tension to at least one of these sheets 11, 12 by adhesive (not shown).

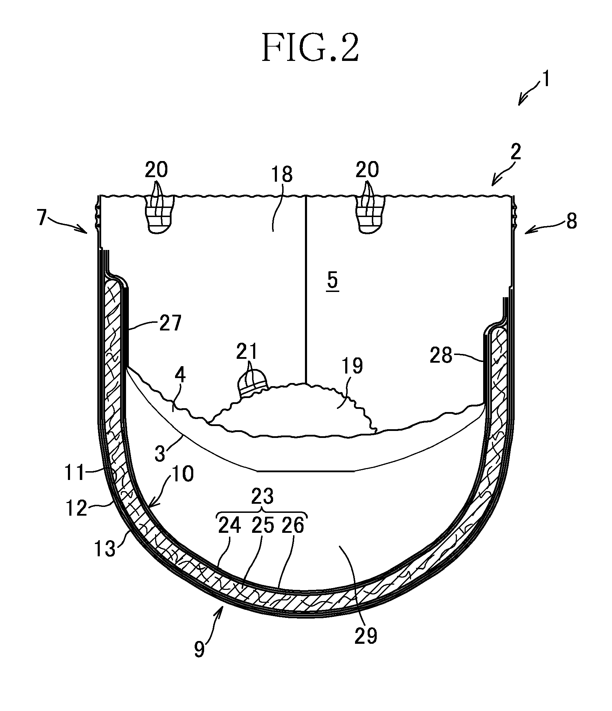

Fig. 2 is a sectional view taken along a line II-II in Fig. 1. As shown in Fig. 2, the chassis 2 is provided on the inner side 5 facing the wearer's skin with a liquid-absorbent structure 10, closer than this liquid-absorbent structure 10 to the wearer's skin with the barrier sheet 3 and further closer than the barrier sheet 3 to the wearer's skin with the leak-barrier cuffs 4. The liquid-absorbent structure 10 is provided so as to extend between the front and rear waist regions 7, 8 in the longitudinal direction Y at least across the crotch region. This liquid-absorbent structure 10 contains a liquid-absorbent panel 23 provided on the inner side of the innerside sheet 11.

The liquid-absorbent panel 23 comprises a liquid-absorbent core 25 wrapped with a liquid-absorbent and spreadable sheet 24 such as tissue paper and an inner sheet 26 wrapping the liquid-absorbent and spreadable sheet 24. The liquid-absorbent panel 23 has its side facing the wearer's clothes covered with the leak-barrier sheet 13 provided on the inner side of the outerside sheet 12 and serving to prevent bodily fluids once absorbed by the core 25 from getting out of the diaper 1. Alternatively, it is possible to bond the leak-barrier sheet 13 directly to the bottom surface of the liquid-absorbent panel 23.

The barrier sheet 3 is bonded in the vicinity of its front and rear ends 27, 28 to an inner sheet 26 by adhesive or sealing. The front end 27 and the rear end 28 respectively lie in the front waist region 7 and the rear waist region 8. In the pants-shaped state as illustrated, the crotch region 9 bows and the barrier sheet 3 is spaced upward from the inner sheet 26 under contraction of the barrier sheet-biasing elastic members as will be described later in more detail. The barrier sheet 3 is bonded in the vicinity of its front and rear ends 27, 28 to the inner sheet 26 so that the barrier sheet 3 as a whole looks like a hammock put up and a void space 29 is defined between the barrier sheet 3 and the liquid-absorbent panel 23. The leak-barrier cuffs 4 are lifted up apart from the barrier sheet 3 under the effect of the cuff-biasing elastic members as will be described later in more detail so as to form a wall extending in the longitudinal direction Y.

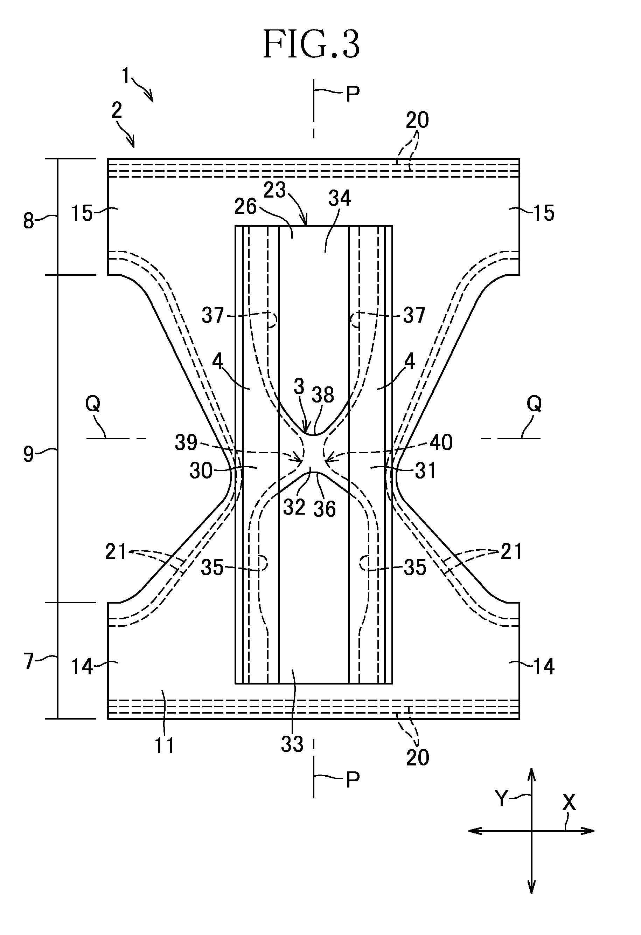

Fig. 3 is a plan view of the diaper 1 as developed in the longitudinal direction Y and the transverse direction X until the front waist region 7, the rear waist region 8 and the crotch region 9 become flush after the front and rear waist regions 7, 8 have been disjoined from each other along respective arrays of the joints as seen in Fig. 1. Obviously, such flatly developed state of the diaper 1 as shown is assumed that the contractile force of the respective elastic members is kept inactivated. The diaper 1 has a longitudinal center line P-P bisecting a dimension of the diaper 1 in the transverse direction X and a transverse center line Q-Q bisecting a dimension of the diaper 1 in the longitudinal direction Y and is bilaterally-symmetric about the longitudinal center line P-P.

The chassis 2 is substantially concave shaped curved inwardly and the liquid-absorbent panel 23 is rectangular wherein the latter is laminated on the innerside sheet 11 of the chassis 2. The barrier sheet 3 has lateral zones 30, 31 opposed to each other in the transverse direction X and a middle zone 32 by the intermediary of which the lateral zones 30, 31 are contiguous to each other wherein the middle zone is on the crotch region 9. The lateral zones 30, 31 and the middle zone 32 cooperate together to form front and rear through-holes 33, 34. The front through-hole 33 extends from the middle zone 32 toward the front waist region 7, substantially describing a U-shape and the rear through-hole 34 extends from the middle zone 32 toward the rear waist region 8, substantially describing a U-shape. The front through-hole 33 is defined by inner side edges 35 respectively extending the lateral zones 30, 31 and a closed curved edge 36 by the intermediary of which the inner side edges 30, 31 are contiguous to each other. The rear through-hole 34 is defined by inner edges 37 of the respective lateral zones 30, 31 and a closed curved edge 38 by the intermediary of which the inner side edges 37 are contiguous to each other.

The leak-barrier cuffs 4 comprise a pair of sheets bilaterally-symmetric about the longitudinal center line P-P and extend in the longitudinal direction Y wherein these sheets are formed by nonwoven fabric or plastic film and preferably liquid-impervious. Each of the leak-barrier cuffs 4 is configured so as to overlap associated one of the lateral zones 30, 31 of the barrier sheet 3 on the side which is facing the wearer's body. Each of the leak-barrier cuffs 4 has a dimension as measured in the transverse direction X is the same as or larger than a dimension of the associated lateral zone 30 or 31 as measured in the corresponding direction X.

Immediately after the diaper 1 has been put on the wearer's body, the diaper's position is adjusted so that the wearer's external genital is opposed to the front through-hole 33, the anus is opposed to the rear through-hole 34 and the wearer's skin zone defined between the external genital and the anus is opposed to the middle zone 32. More specifically, the closed curved edge 36 of the front through-hole 33 preferably lies aside from the transverse center line Q-Q and closed curved edge 38 of the rear through-hole 34 preferably lies on the transverse center line Q-Q or in the vicinity of the transverse center line Q-Q. With the diaper 1 put on the wearer's body in this manner, the liquid-absorbent panel 23 bows in the longitudinal direction Y and the barrier sheet lifts into contact with the wearer's skin under contraction of the first and second barrier sheet-biasing elastic members 39, 40 to form the void space 29 between the barrier sheet 3 and the liquid-absorbent panel 23 (See Fig. 2). In this way, body waste is guided through the front and rear through-holes 33, 34 in the void space 29 and thereby the wearer's skin is protected from contact with body waste.

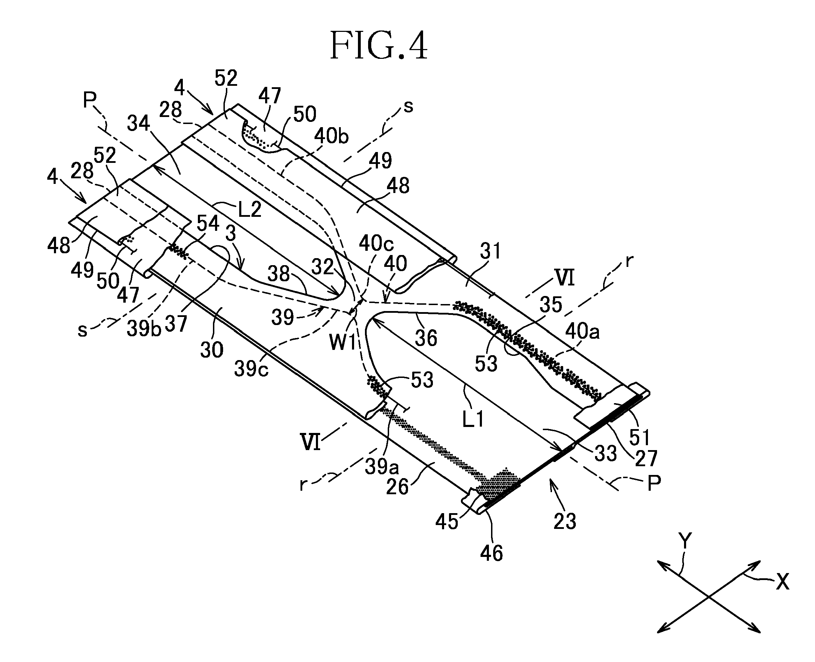

Fig. 4 is a perspective view showing the barrier sheet 3, the leak-barrier cuffs 4 and the liquid-absorbent panel 23 in details wherein the other components are not shown for clarity of illustration. The barrier sheet 3 is provided along the front and rear through-holes 33, 34 with the first and second barrier sheet-biasing elastic members 39, 40 attached under tension thereto. The first and second barrier sheet-biasing elastic members 39, 40 are substantially symmetric about the longitudinal center line P-P, comprise front segments 39a, 40a extending along the front through-hole 33, rear segments 39b, 40b extending along the rear through-hole 34 and convex segments 39c, 40c extending between the front and rear segments along the middle zone 32 toward the longitudinal center line P-P. The first and second barrier sheet-biasing elastic members 39, 40 are spaced from each other by a distance W1 in the transverse direction X minimized along the convex segments 39c, 40c and a distance between the front segments 39a, 40a as well as a distance between the rear segments 39b, 40b is gradually enlarged from the middle zone 32 toward the front and rear ends 27, 28.

The lateral zones 30, 31 of the barrier sheet 3 are folded back onto the liquid-absorbent panel 23 in two-ply fashion. These lateral zones 30, 31 thus folded back in two-ply fashion contain therein the first and second barrier sheet-biasing elastic members 39, 40, respectively, attached thereto by means of adhesive (not shown). The lateral zones 30, 31 of the barrier sheet 3 folded back in this manner are bonded at least along the front and rear ends 27, 28 thereof to the inner sheet 26 of the liquid-absorbent panel 23 by adhesive 45.

The front through-hole 33 of the barrier sheet 3 includes a front center line r-r bisecting a dimension L1 in the longitudinal direction Y from the closed curved edge 36 extending along the middle zone 32 to the front end 27 and the rear through-hole 34 includes a rear center line s-s bisecting a dimension L2 in the longitudinal direction Y from the closed curved edge 38 extending along the middle zone 32 to the rear end 28. It should be appreciated that these dimensions L1 and L2 are the dimensions measured with the elastic members shown in Fig. 4 being inactivated.

Each of the leak-barrier cuffs 4 comprises a three-ply sheet having a Z-shaped cross section. More specifically, the leak-barrier cuff 4 comprises a first layer 46 sandwiched between the liquid-absorbent panel 23 and the chassis 2 and bonded to the innerside sheet 11 of the chassis 2, a second layer 47 folded back from the first layer 46 onto the side of the barrier sheet 3 facing the wearer's skin so as to be placed upon the lateral zone 30 or 31 and a third layer 48 folded back from the second layer 47 onto the side of the second layer 47 facing the wearer's skin. In this manner, the leak-barrier cuff 4 is folded back toward the wearer's skin in the order of the first, second and third layers 46, 47, 48 wherein the first layer 46 and the second layer 47 are placed upon each other by the intermediary of the liquid-absorbent panel 23 and the second layer 47 is placed directly upon the third layer 48. The third layer 48 is folded back onto itself along a side edge 49 thereof so that a cuff-biasing elastic member 50 may be attached thereto. The cuff-biasing elastic member 50 is wrapped with this two-ply side edge 49 and attached under tension so as to extending in the longitudinal direction Y.

At front and rear ends 51, 52 of the leak-barrier cuff 4, the first, second and third layers 46, 48, 49 are bonded one to another and collectively bonded to the innerside sheet 11 of the chassis 2. Under contractile force of the cuff-biasing elastic member 50 exerted on the leak-barrier cuff 4, the second layer 47 and the third layer 48 are unfolded and thereby spaced from the barrier sheet 3 so as to form the wall as seen in Fig. 2. A portion of the second layer 47 and the third layer 48 adapted to be spaced from the barrier sheet 3, i.e., not bonded to the barrier sheet 3 is referred to herein as a free edge.

The barrier sheet 3 and the leak-barrier cuffs 4 are bonded together along front and rear joint zones 53, 54. Each of the front joint zones 53 lies between the associated fixed and free edges so as to overlap each of the front segments 39a, 40a of the first and second barrier sheet-biasing elastic members 39, 40. These front joint zones 53 extend from the front end 27 to the points lying aside from the front center line r-r toward the middle zone 32. Each of the rear joint zones 54 is provided so as to overlap each of the rear segments 39b, 40b of the first and second barrier sheet-biasing elastic members 39, 40 and extends from the rear end 28 to the rear center line s-s. Therefore the rear joint zone 54 has a length dimension as measured in the longitudinal direction Y shorter than the dimension of the front joint zone 53.

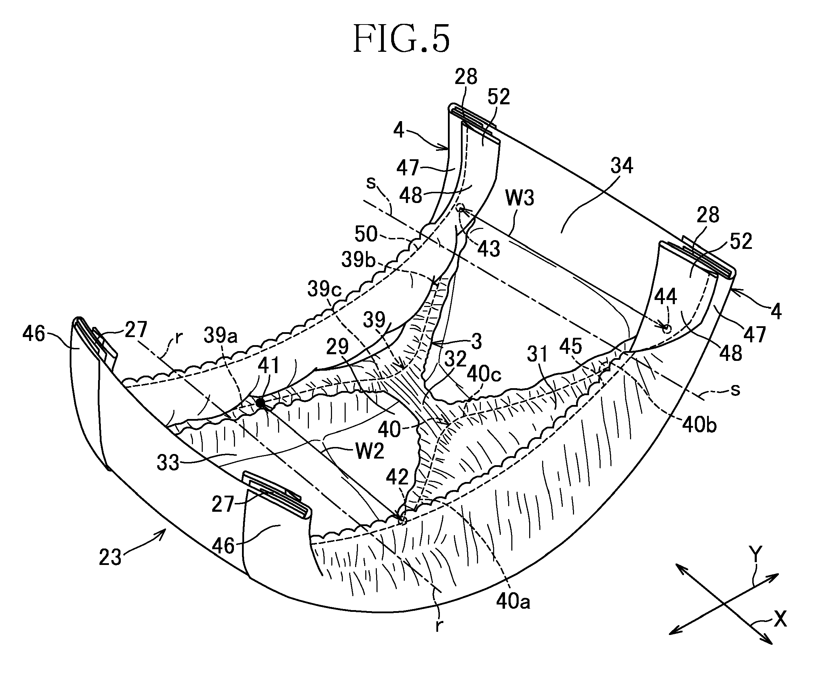

Fig. 5 is a perspective view showing the barrier sheet 3, the leak-barrier cuffs 4 and the liquid-absorbent panel 23. Fig. 5 shows the state in which the liquid-absorbent panel 23 bows as the first and second barrier sheet-biasing elastic members 39, 40 as well as the cuff-biasing elastic members 50 contract. Under contraction of the cuff-biasing elastic members 50, the leak-barrier cuffs 4 are spaced apart from the liquid-absorbent panel 23 so as to rise up. With the cuff-biasing elastic members 50 contracting in this manner, the barrier sheet 3 is pulled upward and outward in the transverse direction X by the intermediary of the front and rear oint zones 53, 54. The front and rear joint zones 53, 54 overlap the first and second barrier sheet-biasing elastic members 39, 40 and, in consequence, the first and second barrier sheet-biasing elastic members 39, 40 are further spaced from each other in the front and rear joint zones 53, 54.

Each of the front joint zones 53 extend from the front end 27 to the points lying aside from the front center line r-r toward the middle zone 32 and correspondingly the first and second barrier sheet-biasing elastic members 39, 40 are stretched more notably in these segments than the remaining segments. Specifically, the front segments 39a, 40a of the first and second barrier sheet-biasing elastic members 39, 40 have front maximally spaced points 41, 42 to define the maximum distance W2 in the respective front joint zones 53 extending from the front end 27 to the points lying aside from the front center line r-r toward the middle zone 32. The rear joint zones 54 extend from the rear end 28 to the rear center line s-s and correspondingly the first and second barrier sheet-biasing members 39, 40 have rear maximally spaced points 43, 44 to define the maximum distance W3 in these rear joint zones 54.

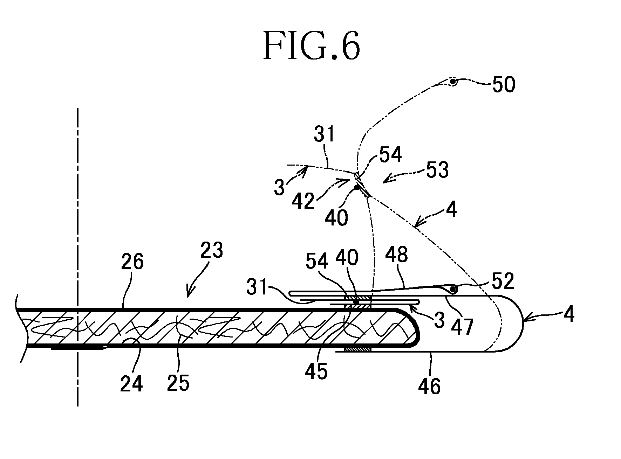

Fig. 6 is a sectional view taken along the line VI-VI in Fig. 4 wherein chain double-dashed lines indicate the second barrier sheet's elastic members 40 and the cuff's elastic member 50 in contracted state thereof. While the lateral zone 31 of the barrier sheet 3 is illustrated herein, it will be appreciated that the description given hereunder is applicable to the lateral zone 30. As will be apparent from Fig. 6, With the elastic members 40, 50 being in contraction state, i.e., with the diaper 1 put on the wearer's body, the second layer 47 and the free edge of the third layer 48 of the leak-barrier cuff 4 are unfolded apart from the liquid-absorbent panel 23. With the leak-barrier cuff 4 unfolded in this manner, the lateral zone 31 bonded at the front joint zone 53 to the second layer 47 is pulled upward and outward in the transverse direction X apart from the liquid-absorbent panel 23. Thus the front through-hole 33 also is pulled as the lateral zone 31 is pulled upward and outward and thereby the open area of the front through-hole 33 is broadened.

According to the present invention, the front maximally spaced points 41, 42 lying in the vicinity of the front through-hole 33 are located aside from the front center line r-r toward the middle zone 32 while the rear maximally spaced points 43, 44 lying in the vicinity of the rear through-hole 34 is located aside from the rear center line r-r toward the rear end 28 (See Fig. 5). In other words, a dimension from the middle zone 32 to a straight line connecting the front maximally spaced points 41, 42 is set to be shorter than a dimension from the middle zone 32 to a straight line connecting the rear maximally spaced points 43, 44. Accordingly, the first and second barrier sheet-biasing elastic members 39, 40 present different configurations between the front through-hole 33 and the rear through-hole 34. In the front through-hole 33, the first and second barrier sheet-biasing elastic members 39, 40 are maximally spaced from each other in the vicinity of the middle zone 32 to prevent these elastic members 39, 40 from coming in contact with the wearer's external genital opposed to this front through-hole 33. As an advantageous result, the front through-hole 33 would not cause undesirable skin irritation and so-called skin trouble.

The rear through-hole 34 is configured so that the first and second barrier sheet-biasing elastic members 39, 40 are maximally spaced from each other on the side of the rear end 28. Such configuration makes it possible to minimize the open area of the rear through-hole 34 on the side of the middle zone 32. It is possible thereby to prevent body waste from coming in contact with the wearer's buttock. In addition, the distance between the rear segments 39b, 40b may be minimized on the side of the middle zone 32 to ensure that the barrier sheet 3 is reliably kept in contact with the wearer's buttock.

It is also possible to set the rear maximally spaced points 43, 44 in the vicinity of the rear end 28. In this case, the barrier sheet 3, the leak-barrier cuffs 4 and the first and second barrier sheet-biasing elastic members 39, 40 are joined together in the vicinity of the rear end 28. With such arrangement, the leak-barrier cuffs 4 are not biased to be raised apart from the barrier sheet 3 and therefore the first and second barrier sheet-biasing elastic members 39, 40 are restricted from moving in the transverse direction X due to the movement of the leak-barrier cuffs 4 which would otherwise occur. Consequently, the lateral zones 30, 31 to which these elastic members 39, 40 are attached are shape-stabilized. In view of the fact that the rear through-hole 34 is formed by the lateral zones 30, 31, the inner side edges 37 of the rear through-hole 34 are also shape-stabilized. Additionally, the open area of the rear through-hole 34 in the vicinity of the middle zone 32 can be minimized.

The rear joint zone 54 has a dimension in the longitudinal direction Y shorter than the corresponding dimension of the front through-hole 53 and, in consequence, the region in which the leak-barrier cuffs 4 are bonded to the barrier sheet 3 is correspondingly smaller on the side of the rear joint zone 54 than on the side of the front joint zone 53. In other words, the region in which the leak-barrier cuffs 4 are spaced from the barrier sheet 3 is correspondingly larger on the side of the rear joint zone 54 than on the side of the front joint zone 53. A void space defined between the barrier sheet 3 and the leak-barrier cuffs 4 assists bodily fluids such as urine to be absorbed and the region in which the leak-barrier cuffs 4 are spaced from the barrier sheet 3 enlarged in this manner serves to enhance the absorption capacity of the diaper 1. Accordingly, urine leak which would otherwise occur along the wearer's buttock and/or dorsal region can be effectively prevented.

The joint zone 53 may be formed by widely used rubber-based hot melt adhesive. It is also possible to form the joint zone 53 by use of widely used means such as ultrasonic or heat sealing technique. While the barrier sheet 3 and each of the leak-barrier cuffs 4 are respectively formed by a single sheet in the illustrated embodiment, it is possible to form the sheet 3 or the cuff 4 by lamination of two sheets. In this case, the barrier sheet-biasing elastic members 39, 40 and cuff-biasing elastic members 50 can be attached so as to sandwich them between these two sheets.

While the leak-barrier cuffs 4 participate in maximizing the distance between the first and second barrier sheet-biasing elastic members 39, 40 according to the illustrated embodiment, it is possible, for example, to locally tuck the lateral zones of the barrier sheet 3 so that these tucked portions of the lateral zones may be pulled outward in the transverse direction X and thereby the distance between the first and second barrier sheet-biasing elastic members 39, 40 may be maximized. Alternatively, the barrier sheet 3 may be bonded to the component other than the leak-barrier cuffs 4, for example, to the innerside sheet 11 to maximize the distance between the first and second barrier sheet-biasing elastic members 39, 40. In this case, the lateral zones 30, 31 of the barrier sheet 3 may be bonded to portions of the innerside sheet 11 lying outside the lateral zones 30, 31 as viewed in the transverse direction X to broaden the distance between the first and second barrier sheet-biasing elastic members 39, 40.

The various components constituting the diaper 1 may be formed from stock materials widely used in the related field of art. For example, the barrier sheet 3 and the leak-barrier cuffs 4 may be formed of a liquid-impervious and an air-permeable nonwoven fabric, the innerside and outerside sheets 11, 12 may be formed of an air-permeable nonwoven fabric, the leak-barrier sheet 13 may be formed of a plastic film, and the liquid-absorbent panel 23 may be formed from a mixture of fluff pulp and super-absorbent polymer particles.

### <Second Embodiment>

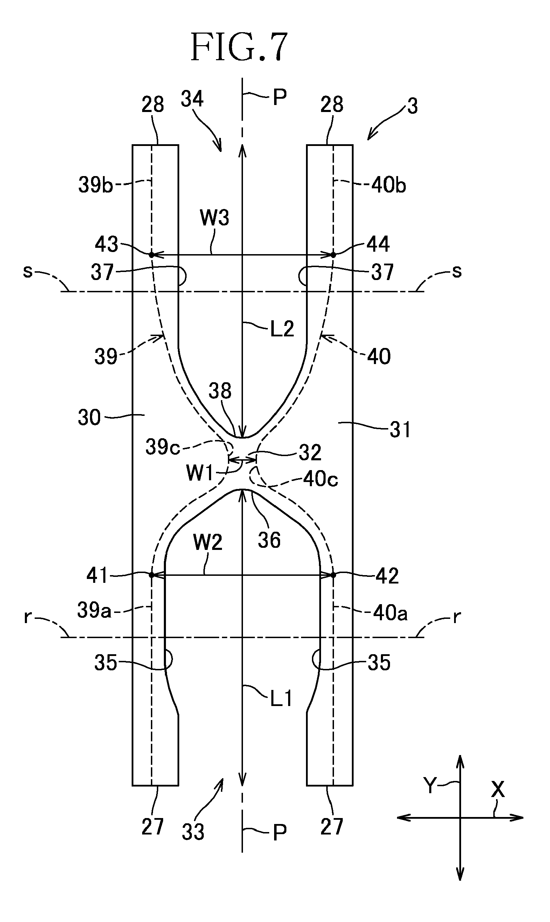

Fig. 7 illustrates a second embodiment of the present invention. The second embodiment is similar to the first embodiment except that the diaper 1 is not provided with the leak-barrier cuffs and the first and second barrier sheet-biasing elastic members are attached in the manner different from the manner used in the first embodiment. The other components are similar to those in the first embodiment and will not be repetitively described.

Fig. 7 particularly illustrates details of the barrier sheet 3. In the state illustrated herein, the barrier sheet 3 is free from a contractile force of the first and second barrier sheet-biasing elastic members 39, 40. As illustrated, the first and second barrier sheet-elastic members 39, 40 extend along the front and rear through-holes 33, 34. The first and second barrier sheet-biasing elastic members 39, 40 are provided in a manner that the front segments 39a, 40a thereof are maximally spaced from each other in the region defined between the middle zone 32 and the front center line r-r and the rear segments 39b, 40b thereof are maximally spaced from each other in the region defined between the rear end 28 and the rear center line s-s.

When the diaper having such barrier sheet 3 is put on the wearer's body, the first and second barrier sheet-biasing elastic members 39, 40 contract but the dimension of the barrier sheet 3 in the transverse direction X is substantially not affected thereby. Thus, the front maximally spaced points 41, 42 between which the front segments 39a, 40a are maximally spaced from each other are formed in a region defined between the middle zone 32 and the front center line r-r and the rear maximally spaced points 43, 44 between which the rear segments 39b, 40b are maximally spaced from each other are formed in a region defined between the rear end 28 and the rear center line s-s.

According to this second embodiment, no separate members are needed to broaden the distance between the first and second barrier sheet-biasing elastic members 39, 40 and therefore the number of parts can be reduced and thereby the cost can be correspondingly reduced.

## Claims

1. An absorbent article having a longitudinal direction and a transverse direction, a side facing a wearer's skin and a side facing said wearer's clothes, a front waist region, a rear waist region and a crotch region extending between said front and rear waist regions, and comprising a chassis provided in said crotch region with a liquid-absorbent structure and a barrier sheet provided in said crotch region on a side of said chassis facing said wearer's skin and so as to be movable apart from said chassis, said absorbent article being **characterized in that:**
said barrier sheet having lateral zones extending in said longitudinal direction and opposed to each other in said transverse direction, a middle zone by the intermediary of which said lateral zones are contiguous to each other, a front through-hole formed in said front waist region so as to be bordered by said middle zone, a rear through-hole formed in said rear waist region so as to be bordered by said middle zone, front and rear ends extending in said transverse direction and opposed to each other in said longitudinal direction, and first and second barrier sheet-biasing elastic members opposed to each other in said transverse direction and extending in said longitudinal direction and attached under tension to said barrier sheet in a substantially symmetric relationship about a longitudinal center line bisecting a dimension of said barrier sheet in said transverse direction wherein said front and rear ends are permanently bonded to said side of said chassis facing said wearer's skin and a void space is formed between said barrier sheet and said liquid-absorbent structure as said liquid-absorbent structure bows from said crotch region toward said front and rear waist regions;
said front and rear through-holes are defined by said lateral zones and said middle zone and adapted to guide body waste into said void space;
said first and second barrier sheet-biasing elastic members comprise front segments extending along said front through-hole and rear segments extending along said rear through-hole;
assumed that said front and rear waist regions and said crotch region are forced into a substantially coplanar relationship, said front segments of said first and second barrier sheet-biasing elastic members are maximally spaced from each other in said transverse direction in a region defined aside from a front center line bisecting a dimension of said front through-hole in said longitudinal direction toward said middle zone; and said rear segments are maximally spaced from each other in said transverse direction in a region defined aside from a rear center line bisecting a dimension of said rear through-hole in said longitudinal direction toward said rear end.

2. The absorbent article according to Claim 1, wherein:
said absorbent article further comprises a pair of leak-barrier cuffs provided on a side of said barrier sheet facing said wearer's skin so as to extend in said longitudinal direction and to be opposed to each other in said transverse direction; each of said leak-barrier cuffs has a fixed edge joined to said chassis, a free edge not joined to said chassis so as to be movable apart from said chassis and cuff-biasing elastic member attached under tension to said free edge and causing said leak-barrier cuff to be spaced from said chassis; and said barrier sheet and said leak-barrier cuffs are joined together in front joint zones lying between said fixed edges and said free edges opposed one to another in said transverse direction and overlapping said front segments of said first and second barrier sheet-biasing elastic members and said front joint zones extend intermittently or continuously from said front end of said barrier sheet to points at which said first and second barrier sheet-biasing elastic members are maximally spaced from each other in said transverse direction.

3. The absorbent article according to Claim 2, wherein said barrier sheet and said leak-barrier cuffs are jointed together at said rear end of said barrier sheet by means of rear joint zones and said rear segments of said first and second barrier sheet-biasing elastic members are joined together in the vicinity of said rear end of said barrier sheet so that said first and second barrier sheet-biasing elastic members are maximally spaced from each other in said transverse direction in said joint zones.

4. The absorbent article according to any one of Claims 1 through 3, wherein a dimension measured from said middle zone to a straight line connecting points at which front segments of said first and second barrier sheet-biasing elastic members are maximally spaced from each other is set to be shorter than a dimension measured from said middle zone to a straight line connecting points at which rear segments of said first and second barrier sheet-biasing elastic members are maximally spaced from each other.
